# FASCICULE DE BREVET EUROPEEN

(11) **EP 2 026 796 B1**
(45) Date de publication et mention de la délivrance du brevet: **09.11.2016**
(21) Numéro de dépôt: 07788875.8
(22) Date de dépôt: 12.06.2007
(51) Int. Cl.: A61K 31/36, A61K 31/519, A61K 45/06, A61P 9/12

(54) **UTILISATION D'UN INHIBITEUR DE VASOPEPTIDASE POUR LE TRAITEMENT DE L'HYPERTENSION ARTERIELLE PULMONAIRE**
VERWENDUNG EINES VASOPEPTIDASE-HEMMERS ZUR BEHANDLUNG VON LUNGEN-ARTERIEN-HOCHDRUCK
USE OF A VASOPEPTIDASE INHIBITOR FOR THE TREATMENT OF PULMONARY ARTERIAL HYPERTENSION

(30) Priorité: 13.06.2006 FR 0605250
(43) Date de publication de la demande: 25.02.2009
(73) Titulaire: BIOPROJET, 75003 Paris (FR)
(72) Inventeur: SCHWARTZ, Jean-Charles, 75014 Paris (FR); LECOMTE, Jeanne-Marie, 75003 Paris (FR)
(74) Mandataire: Lavoix
(86) Numéro de dépôt international: PCT/FR2007/000970
(87) Numéro de publication internationale: WO 2007/144501

(56) Documents cités:
- WO-A-2005/102317
- WO-A-2006/126082
- US-A1- 2004 220 186
- "The Merck Index, 13th Edition" 2001, MERCK & CO., INC. , WHITEHOUSE STATION, NJ, USA , XP002415987 pages THER-30, colonne de droite, alinéa 2
- BIN YAP LOK ET AL: "Natriuretic peptides, respiratory disease, and the right heart" CHEST, vol. 126, no. 4, octobre 2004 (2004-10), pages 1330-1336, XP002415985 ISSN: 0012-3692

## Description

L'hypertension artérielle pulmonaire (HTAP) est une maladie mortelle caractérisée par des lésions intimales, une hypertrophie médiale et un épaississement des artères pulmonaires précapillaires. L'augmentation de la post-charge qui en résulte aboutit au développement de l'hypertrophie ventriculaire droite et d'une insuffisance cardiaque droite. Les mécanismes pathogènes sous-jacents semblent être une augmentation de la tonalité vasomotrice et la transformation chronique des vaisseaux précapillaires de résistance.

Le traitement de cette maladie sévère mais relativement rare a été amélioré pendant la dernière décennie par l'utilisation de drogues provoquant la vasodilatation pulmonaire aiguë, par exemple les dérivés de prostacycline, les antagonistes du récepteur de l'endothéline ou les inhibiteurs de phosphodiestérase. Néanmoins, chacun de ces traitements a des inconvénients : les agonistes de prostacycline présentent des demi-vies relativement courtes et doivent être administrés par injections continues, tandis que les autres sont moins efficaces et/ou montrent une toxicité pour le foie (Bull. T. R., Semin. Crit. Care Med., 2005, 26, 425). Par conséquent, ces diverses approches thérapeutiques sont souvent combinées mais doivent toutefois être améliorées.

Le fasidotril ou (S, S)-2(2-acétylsulfanylméthyl-3-benzo[1,3]dioxo-5-yl-propionyl amino)propionate de benzyle est le prototype d'une nouvelle classe de médicaments cardiovasculaires, souvent désignés comme "les inhibiteurs de vasopeptidase", qui combinent dans la même molécule une puissante activité inhibitrice de deux métallopeptidases, de l'enzyme de conversion de l'angiotensine (ACE, EC 3.4.15.1) et de la néprilysine (EC 3.4.24.11) impliquées dans le métabolisme des messagers peptidiques cardiovasculaires.

On a démontré que les inhibiteurs de vasopeptidase sont utiles dans les modèles animaux et les essais cliniques humains pour l'hypertension artérielle et les incidents cardiaques congestifs, deux conditions pathologiques relativement fréquentes qui diffèrent clairement de l'HTAP en termes de pathogénie, lésions, symptômes et traitements reconnus. Ces médicaments n'ont jamais été proposés pour l'utilisation dans l'HTAP, et il existe même des rapports expérimentaux suggérant que l'inhibition de l'ACE peut être inefficace voire préjudiciable dans le modèle animal de cette maladie (Ishikawa et al., J. Intern. Cardiol., 1995, 47, 225 ; Zhou et Lai, J. Appl. Physiol., 1993, 75, 2781).

Bin Yap Lok et al Chest, 126, 4, 2004 suggère l'utilisation d'inhibiteurs de l'endopeptidase neutre (NEP) en tant qu'adjuvants possibles de principes actifs de type peptide natriurétique possédant une activité propre dans l'HTAP.

Les présents inventeurs ont démontré de façon inattendue que, d'une part, les inhibiteurs de vasopeptidase utilisés seuls sont efficaces dans l'HTAP et que, d'autre part, les combinaisons d'un inhibiteur de vasopeptidase avec un traitement reconnu de l'HTAP sont extrêmement efficaces en ralentissant le développement de la maladie.

Selon un premier objet, la présente invention concerne donc un inhibiteur de vasopeptidase à titre de seul agent actif ou en combinaison avec un inhibiteur de phosphodiesterase pour utilisation dans le traitement et/ou la prévention de l'HTAP, tel que l'inhibiteur de vasopeptidase est le fasidotril ou ses sels pharmaceutiquement acceptables, et l'inhibiteur de phosphodiesterase est le sildenafil.

Selon un second objet, la présente invention concerne également les combinaisons d'un inhibiteur de vasopeptidase et d'un inhibiteur de phosphodiesterase avec un véhicule pharmaceutiquement acceptable, tel que l'inhibiteur de vasopeptidase est le fasidotril ou ses sels pharmaceutiquement acceptables et l'inhibiteur de phosphodiesterase est le sildenafil.

Selon un autre objet, la présente invention concerne également une combinaison selon l'invention pour son utilisation pour le traitement et/ou la prévention de l'HTAP.

La présente invention concerne également les médicaments comprenant le fasidotril ou ses sels pharmaceutiquement acceptables à titre de seul agent actif ou en combinaison avec le sildenafil pour le traitement et/ou la prévention de l'HTAP.

Selon l'invention, les combinaisons permettent l'administration simultanée séparée ou séquencée dans le temps des deux ingrédients actifs.

De préférence, la combinaison selon l'invention se présente sous une forme adaptée à l'administration par voie orale.

La combinaison selon l'invention démontre un effet synergique.

Selon un aspect préféré, la combinaison selon l'invention contient les deux ingrédients actifs dans la même dose unitaire.

L'inhibiteur de vasopeptidase est le fasidotril ou ses sels pharmaceutiquement acceptables.

L'inhibiteur de phosphodiestérase est l' inhibiteur de PDE-5, sildénafil (Viagra®).

De préférence, l'inhibiteur de vasopeptidase se présente sous une forme adaptée à l'administration par voie orale. Généralement, ledit inhibiteur de vasopeptidase est administré une à trois fois par jour, de préférence deux fois par jour, à des doses comprises entre 10 et 200 mg, de préférence environ 100 mg deux fois par jour.

De préférence, l'inhibiteur de PDE-5 se présente sous une forme adaptée à l'administration par voie orale, typiquement sous forme de comprimés pelliculés. Généralement, ledit inhibiteur de PDE-5 est administré une à trois fois par jour, de préférence deux fois par jour, à des doses comprises entre 10 et 200mg.

La quantité de chaque composant administré est déterminée par les médecins traitants en tenant compte de l'étiologie et de la gravité de la maladie, l'état et l'âge du malade, la puissance de chaque composant et autres facteurs.

Plus généralement, les médicaments selon l'invention peuvent être présentées sous des formes destinées à l'administration par voie parentérale, orale, rectale, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalino-terreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1000 mg) en fonction de l'indication thérapeutique et de la voie d'administration, ainsi que de l'âge et du poids du sujet.

"Formulations adaptées pour une administration orale" signifie des formulations qui sont sous une forme adaptée pour être administrées oralement à un patient. Les formulations peuvent être présentées comme unités discrètes telles que les capsules, les cachets ou les comprimés, chacun contenant une quantité prédéterminée de l'ingrédient actif ; comme une poudre ou des granulés ; comme une solution ou une suspension dans un liquide aqueux ou un liquide non aqueux ; ou comme une émulsion liquide huile-dans-l'eau ou une émulsion liquide eau-dans-l'huile. L'ingrédient actif peut aussi être présenté sous la forme d'un bol, d'un électuaire ou d'une pâte. Dans les formes pharmaceutiques solides, le composé utile selon l'invention est mélangé avec au moins un excipient ordinaire inerte (ou véhicule) tel que le citrate de sodium ou le phosphate dicalcique ou (a) des excipients ou diluants, comme par exemple les amidons, le lactose, le saccharose, le glucose, le mannitol et l'acide silicique, (b) des liants comme par exemple le carboxyméthylcellulose, les alignates, la polyvinylpyrrolidone, le saccharose et l'acacia, (c) des humectants, comme par exemple le glycérol, (d) des délitants, comme par exemple l'agar-agar, le carbonate de calcium, le fécule de pomme de terre ou de manioc, l'acide alginique, certains silicates complexes et carbonate de sodium, (e) des retardateurs de solution comme par exemple la paraffine, (f) des accélérateurs d'absorption comme par exemple des composés d'ammonium quaternaire, (g) des agents mouillants comme par exemple l'alcool cétylique et le monostéarate de glycéryle, (h) des adsorbants comme par exemple du kaolin et de la bentonite, (i) des lubrifiants comme par exemple le talc, le stéarate de calcium, le stéarate de magnésium, les polyéthylèneglycols solides, le sulfate sodique de lauryle, (j) des agents opacifiants, (k) des tampons et des agents qui libèrent en différé le(s) composé(s) utile(s) selon l'invention dans un certaine partie du tractus intestinal.

"Formulations adaptées pour une administration parentérale" signifie des formulations qui sont sous une forme adaptée pour être administrées par voie parentérale à un patient. Les formulations sont stériles et comprennent les émulsions, les suspensions, les solutions d'injection aqueuses et non aqueuses, qui peuvent contenir des agents de suspension et des agents épaississants et des anti-oxydants, des tampons, des bactériostatiques et des solutés qui rendent la formulation isotonique, et ont un pH ajusté de manière adaptée, avec le sang du receveur voulu.

"Formulations adaptées pour une administration rectale" signifie des formulations qui sont sous une forme adaptée pour être administrées par voie rectale à un patient. La formulation est de préférence sous la forme de suppositoires qui peuvent être préparés en mélangeant les composés utiles selon la présente invention avec des excipients ou des véhicules non irritants adaptés tels que le beurre de cacao, le polyéthylèneglycol, ou une cire de suppositoire, qui sont solides aux températures ordinaires mais liquides à la température corporelle et, ainsi, fondus dans le rectum ou la cavité vaginale, et libèrent le composant actif.

Pour les administrations par voie percutanée ou permuqueuse, la formulation peut se présenter comme une pommade topique, des baumes, des poudres, des sprays et des inhalants, des gels (à base d'eau ou d'alcool), des crèmes, comme il est généralement connu dans la technique, ou incorporée dans une base de matrice pour une application dans un timbre, qui permettrait une libération contrôlée du composé par la barrière transdermique.

"Patient" comprend à la fois un humain et d'autres mammifères.

"Composition pharmaceutique" signifie une composition comprenant un composé de formule I et au moins un composant choisi dans le groupe comprenant les transporteurs, diluants, adjuvants, excipients, ou véhicules pharmaceutiquement acceptables, tels que les agents de conservation, les charges, les agents désintégrants, les agents mouillants, les agents émulsifiants, les agents de suspension, les agents édulcorants, les agents aromatisants, les agents parfumants, les agents antibactériens, les agents antifongiques, les agents lubrifiants, et les agents dispersants, selon la nature du mode d'administration et les formes de dosage. Les exemples d'agents de suspension comprennent les alcools isostéaryle éthoxylés, le polyoxyéthylène sorbitol et les esters de sorbitan, la cellulose microcristalline, le métahydroxyde d'aluminium, la bentonite, l'agar-agar et la tragacanthe, ou des mélanges de ces substances. La prévention de l'action des microorganismes peut être assurée par divers agents antibactériens et antifongiques, par exemple les parabènes, le chlorobutanol, le phénol, l'acide sorbique, et similaires. Il peut aussi être souhaitable de comprendre les agents isotoniques, par exemple les sucres, le chlorure de sodium et similaires. L'absorption prolongée de la forme pharmaceutique injectable peut être effectuée par l'utilisation d'agents retardant l'absorption, par exemple, le monostérate d'aluminium et la gélatine. Les exemples de transporteurs, diluants, solvants ou véhicules adaptés comprennent l'eau, l'éthanol, les polyols, leurs mélanges adaptés, les huiles végétales (telles que l'huile d'olive) et les esters organiques injectables tels que l'éthyl oléate. Les exemples d'excipients comprennent le lactose, le sucre de lait, le citrate de sodium, le carbonate de calcium, le phosphate phosphate dicalcique. Les exemples d'agents désintégrants comprennent l'amidon, les acides alginiques et certains silicates complexes. Les exemples de lubrifiants comprennent le stéarate de magnésium, le sodium lauryl sulfate, le talc, ainsi que des polyéthylèneglycols de haute masse moléculaire.

"Pharmaceutiquement acceptable" signifie que c'est, dans la portée d'un jugement médical valable, adapté pour une utilisation en contact avec les cellules des humains et des animaux inférieurs sans toxicité, irritation, réponse allergique indue et similaires, et sont proportionnés à un rapport avantage/risque raisonnable.

Les ingrédients actifs peuvent être utilisés sous forme libre ou de leur sels pharmaceutiquement acceptables.

L'expression "sels pharmaceutiquement acceptables" fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valérate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, méthanesulfonates, éthane-sulfonates, benzènesulfonates, p-toluènesulfonates, cyclohexyl sulfamates et quinateslaurylsulfonate, et analogues. (Voir par exemple S.M. Berge et al. "Pharmaceutical Salts " J. Pharm. Sci, 66 :p.1-19 (1977) qui est incorporé ici en référence). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les aminés qui sont souvent utilisées en chimie médicinale en raison de leur faible toxicité et de leur acceptabilité pour l'usage médical : ammoniac, éthylènediamine, N-méthyl-glucamine, lysine, arginine, ornithine, choline, N,N'-dibenzyléthylènediamine, chloroprocaïne, diéthanolamine, procaïne, N-benzylphénéthylamine, diéthylamine, pipérazine, tris(hydroxyméthyl)-amino-méthane, hydroxyde de tétraméthylammonium, triéthylamine, dibenzylamine, éphénamine, dehydroabiétylamine, N-éthylpipéridine, benzylamine, tétra-méthylammonium, tétraéthylammonium, méthylamine, diméthylamine, triméthyl-amine, éthylamine, acides aminés de base, par exemple lysine et arginine, et dicyclohexylamine, et analogues.

Les exemples suivants sont donnés à titre illustratif de la présente invention.

### Exemple 1

L'efficacité à long terme du fasidotril a été étudiée sur un modèle reconnu de l'HTAP, chez des rats recevant une injection de l'alcaloïde toxique monocrotaline (Rosenberg et Rabinovitch, Am. J. Physiol., 1988, 255, 484). Après une injection unique de la toxine (60 mg/kg, s.c.), l'endothélium artériel pulmonaire est lésé, et les animaux développent une hypertrophie du muscle lisse de l'artère pulmonaire avec une hypertension pulmonaire sévère et une hypertrophie ventriculaire droite.

Un groupe de 50 rats de type Wistar pesant environ 300 g a été utilisé, parmi lequel 10 ont servi de contrôle tandis que 40 ont reçu une dose de 60 mg/kg de monocrotaline s.c. Ces derniers ont été divisés en deux groupes de 20 animaux : le premier groupe a été alimenté avec une alimentation standard de laboratoire tandis que le second groupe a été alimenté avec une alimentation contenant le fasidotril à une concentration telle que les rats ont reçu une dose d'environ 200 mg/kg/jour.

Après un mois, la mortalité était de 45 % dans le groupe "monocrotaline seul" mais de seulement 20 % dans le groupe "monocrotaline/fasidotril". De plus, la monocrotaline a provoqué une hypertrophie droite du coeur marquée, avec une augmentation de + 140 % et + 74 % du poids de l'auricule et du ventricule droits respectivement, tandis que les valeurs correspondantes dans le groupe "fasidotril" étaient seulement de + 57 % et + 50 %.

Il apparaît donc clairement de ces données que le traitement avec le fasidotril a ralenti environ de moitié la vitesse de développement de la maladie dans ce modèle d'HTAP.

### Exemple 2

Le fasidotril est associé au sildénafil, un inhibiteur de la phosphodiestérase 5, qui a été considéré comme actif dans le modèle d'HTAP (Schermuly et al., Amer. J. Resp. Crit. Care Med. 2004, 169, 39) :
Un groupe de rats Wistar de 250-275g reçoit par voie sous-cutanée de la monocrotaline (60 mg/kg,s.c.) puis est traité pendant 1 mois par fasidotrilate (100mg/kg ,b.i.d.), sildenafil (5mg/kg, b.i.d.) ,une association de fasidotrilate et sildenafil, ou du vehicule (témoins).Les rats sont pesés au cours des traitements puis sacrifiés à leur terme. L'hypertophie du coeur droit évaluée, notamment par dissection et pesée.

Chacun des traitements séparé parait prévenir partiellement le développement de l'hypertrophie par rapport aux témoins mais leur association conduit à une protection quasi-totale. Ceci indique, pour la première fois l'intérêt synergique de cette association.

## Revendications

1. Inhibiteur de vasopeptidase à titre de seul agent actif ou en combinaison avec un inhibiteur de phosphodiestérase pour utilisation dans le traitement et/ou la prévention de l'hypertension artérielle pulmonaire (HTAP), tel que l'inhibiteur de vasopeptidase est le fasidotril ou ses sels pharmaceutiquement acceptables, et l'inhibiteur de phosphodiestérase est le sildénafil.

2. L'inhibiteur de vasopeptidase fasidotril ou ses sels pharmaceutiquement acceptables pour utilisation selon la revendication 1 pour administration par voie orale.

3. L'inhibiteur de vasopeptidase fasidotril ou ses sels pharmaceutiquement acceptables pour utilisation selon l'une quelconque des revendications 1 à 2, pour l'administration de 10 à 200 mg dudit inhibiteur de vasopeptidase.

4. L'inhibiteur de vasopeptidase fasidotril ou ses sels pharmaceutiquement acceptables en combinaison avec un inhibiteur de phosphodiestérase sildénafil pour utilisation selon l'une quelconque des revendications 1 à 3.

5. Combinaison d'un inhibiteur de vasopeptidase et d'un inhibiteur de phosphodiestérase avec un véhicule pharmaceutiquement acceptable, telle que tel que l'inhibiteur de vasopeptidase est le fasidotril ou ses sels pharmaceutiquement acceptables et l'inhibiteur de phosphodiestérase est le sildénafil.

6. Combinaison selon la revendication 5 permettant l'administration simultanée séparée ou séquencée dans le temps des deux ingrédients actifs.

7. Combinaison selon l'une quelconque des revendications 5 à 6 telle qu'elle permet l'administration de 10 à 200 mg de l'inhibiteur de vasopeptidase et de 10 à 200 mg de l'inhibiteur de PDE-5.

8. Combinaison selon l'une quelconque des revendications 5 à 7 pour utilisation dans le traitement et/ou la prévention de l'HTAP.

9. Médicament comprenant le fasidotril ou ses sels pharmaceutiquement acceptables à titre de seul agent actif pour utilisation dans le traitement et/ou la prévention de l'HTAP.

10. Médicament comprenant le fasidotril ou ses sels pharmaceutiquement acceptables pour utilisation dans le traitement et/ou la prévention de l'HTAP en combinaison avec l'inhibiteur de phosphodiestérase sildénafil.

## Patentansprüche

1. Vasopeptidaseinhibitor als einziger aktiver Wirkstoff oder in Kombination mit einem Phosphodiesteraseinhibitor für die Verwendung bei der Behandlung und/oder der Verhinderung einer pulmonalen arteriellen Hypertonie (HTAP), derart, dass der Vasopeptidaseinhibitor Fasidotril oder seine pharmazeutisch akzeptable Salze ist und der Phosphodiesteraseinhibitor Sildenafil ist.

2. Fasidotril-Vasopeptidaseinhibitor oder seine pharmazeutisch akzeptable Salze für die Verwendung nach Anspruch 1 für die orale Verabreichung.

3. Fasidotril-Vasopeptidaseinhibitor oder seine pharmazeutisch akzeptable Salze für die Verwendung nach einem der Ansprüche 1 bis 2 für die Verabreichung von 10 bis 200 mg des Vasopeptidaseinhibibitors.

4. Fasidotril-Vasopeptidaseinhibitor oder seine pharmazeutisch akzeptable Salze in Kombination mit dem Sildenafil-Phospodiesteraseinhibitor für die Verwendung nach einem der Ansprüche 1 bis 3.

5. Kombination aus einem Vasopeptidaseinhibitor und einem Phosphodiesteraseinhibitor mit einer pharmazeutisch akzeptablen Trägersubstanz, derart, dass der Vasopeptidaseinhibitor Fasidotril oder seine pharmazeutisch akzeptablen Salze ist und der Phosphodiesteraseinhibitor Sildenafil ist.

6. Kombination nach Anspruch 5, die die gleichzeitige oder zeitlich aufeinander folgende Verabreichung von zwei aktiven Ingredienzien ermöglicht.

7. Kombination nach einem der Ansprüche 5 bis 6, derart, dass sie die Verabreichung von 10 bis 200 mg des Vasopeptidaseinhibitors und von 10 bis 200 mg des PDE-5-Inhibitors ermöglicht.

8. Kombination nach einem der Ansprüche 5 bis 7 für die Verwendung bei der Behandlung und/oder der Verhinderung von HTAP.

9. Medikament, das Fasidotril als einzigen aktiven Wirkstoff für die Verwendung bei der Behandlung und/oder der Verhinderung von HTAP enthält.

10. Medikament, das Fasidotril für die Verwendung bei der Behandlung und/oder der Verhinderung von HTAP in Kombination mit dem Phosphodiesteraseinhibitor Sildenafil enthält.

## Claims

1. A vasopeptidase inhibitor as the only active agent or in combination with a phosphodiesterase inhibitor for use for the treatment and/or prevention of pulmonary arterial hypertension (PAH), wherein the vasopeptidase inhibitor is fasidotril or its pharmaceutically acceptable salts, and the phosphodiesterase inhibitor is sildenafil.

2. The vasopeptidase inhibitor fasidotril or its pharmaceutically acceptable salts for use according to claim 1 for administration by the oral route.

3. The vasopeptidase inhibitor fasidotril or its pharmaceutically acceptable salts for use according to claim 1 or 2 for administration of from 10 to 200mg of the vasopeptidase inhibitor.

4. The vasopeptidase inhibitor fasidotril or its pharmaceutically acceptable salts in combination with the phosphodiesterase inhibitor sildenafil for useaccording to anyone of claims 1 to 3.

5. Combination of a vasopeptidase inhibitor and a phosphodiesterase inhibitor with a pharmaceutically acceptable vehicle, wherein the vasopeptidase inhibitor is fasidotril or its pharmaceutically acceptable salts and the phosphodiesterase inhibitor is sildenafil.

6. Combination according to claim 5enabling the two active ingredients to be administered in a separate manner, a simultaneous manner or in a manner sequential in time.

7. Combination according to any one of claims 5 or 6 such that it allows the administration of from 10 to 200 mg of the vasopeptidase inhibitor and from 10 to 200 mg of the PDE-5 inhibitor.

8. Combination according to any one of claims 5 to 7 for use for the treatment and/or prevention of PAH.

9. Medicament comprising fasidotril as the only active agent for use for the treatment and/or prevention of PAH.

10. Medicament comprising fasidotril for use for the treatment and/or prevention of PAH in combination with the phosphodiesterase inhibitor sildenafil.
